# EUROPEAN PATENT SPECIFICATION

(11) **EP 2 054 420 B1**
(45) Date of publication and mention of the grant of the patent: **22.06.2011**
(21) Application number: 07793796.9
(22) Date of filing: 01.08.2007
(51) Int. Cl.: C07D 513/04, A61K 31/4188, A61P 27/06

(54) **BENZIMIDAZO[1,2-C][1,2,3]THIADIAZOL-7-SULFONAMIDES AS INHIBITORS OF CARBONIC ANHYDRASE AND THE INTERMEDIATES FOR PRODUCTION THEREOF**
BENZIMIDAZO[1,2-C][1,2,3]THIADIAZOL-7-SULFONSÄUREAMIDE ALS CARBOANHYDRASEINHIBITOREN UND ZWISCHENPRODUKTE ZU DEREN HERSTELLUNG
BENZIMIDAZO[1,2-C][1,2,3]THIADIAZOL-7-SULFAMIDES EN TANT QU'INHIBITEURS D'UNE ANHYDRASE CARBONIQUE ET LEURS INTERMÉDIAIRES DE PRODUCTION

(30) Priority: 02.08.2006 LT 2006066
(43) Date of publication of application: 06.05.2009
(73) Proprietor: Biotechnologijos Institutas, 02241 Vilnius (LT)
(72) Inventor: MATULIS, Daumantas, 06230 Vilnius (LT); DUDUTIENE, Virginija, 06145 Vilnius (LT); MATULIENE, Jurgita, 06230 Vilnius (LT); MISTINAITE, Lina, 03218 Vilnius (LT)
(74) Representative: Gerasimovic, Liudmila
(86) International application number: PCT/LT2007/000005
(87) International publication number: WO 2008/016288

(56) References cited:
- WO-A-03/013655
- US-A- 5 235 059
- US-A- 5 681 834
- SIGITAS TUMKEVICIUS ET AL: "Synthesis and structure of benzimidazo[1,2-c][1,2,3]thiadiazoles: first examples of a novel ring system" TETRAHEDRON LETTERS, vol. 44, 2003, pages 6635-6638, XP004442819
- V. DUDUTIENE ET AL: "Benzimidazo[1,2-c][1,2,3]thiadiazole-7-su lfonamides as inhibitors of carbonic anhydrase", BIOORGANIC & MEDICINAL CHEMISTRY LETTERS, vol. 17, 5 April 2007 (2007-04-05), pages 3335-3338,
- V. DUDUTIENE ET AL: "Benzimidazo[1,2-c][1,2,3]thiadiazole-7-su lfonamides as inhibitors of carbonic anhydrase" BIOORGANIC & MEDICINAL CHEMISTRY LETTERS, vol. 17, August 2003 (2003-08), pages 3335-3338,

## Description

### Field of the Invention

The present invention describes novel aromatic and heterocyclic sulfonamide derivatives, potentially useful in biomedicine as active ingredients of pharmaceutical preparations because of their ability to inhibit enzymes participating in disease progression: The invention also relates to new intermediate compounds required for the synthesis of target sulfonamides.

The enzymes in this description of the invention include different metal (mostly zinc) ion-possessing proteins, such as carbonic anhydrases and metalloproteinases.

### Background of the Invention

Carbonic anhydrases (CA) are zinc-containing enzymes which catalyze reversible reaction of carbon dioxide hydration. These enzymes participate in essential physiological processes related to respiration, CO₂/bicarbonate transport between lungs and metabolizing tissues, pH and CO₂ homeostasis, electrolite secretion in many tissues/organs, etc. To date there are 15 carbonic anhydrase isozymes identified in humans - 12 catalytically active and 3 inactive, so called carbonic anhydrase related proteins. The 12 active isoforms have different subcellular localization - 5 of them are cytosolic, 4 - membrane bound, 2 mitochondrial and 1 - secreted. There are two major classes of carbonic anhydrase inhibitors: 1) metal-complexing anions; 2) aromatic and heterocyclic inhibitors possessing sulfonamide group. Sulfonamide-class carbonic anhydrase inhibitors are widely used as therapeutic agents for treatment of various diseases, since the 15 carbonic aanhydrase isozymes are widely distributed in most of the cells, tissues and organs where they are responsible for essential physiological functions. Another similar protein class is metalloproteinases, proteolytic enzymes, which are characterized by increased expression during various steps of cancer progression. Sulfonamide inhibitors have a great potencial for the inhibition of metalloproteinases.

Carbonic anhydrases participate in many essential physiological processes, therefore the increased activity or expression of different CA isoforms results in significant pathological outcomes. Therefore the regulation of CA catalytic activity by means of inhibition or activation proposes a therapeutic perspective.

There are several diseases with the characteristic disbalance of the interconversion between carbonic dioxide and bicarbonate resulting in pH alteration, disturbance of ion transport, fluid secretion, etc. A classical example of such a disease is glaucoma. While most often carbonic anhydrase inhibitors are used as antiglaucoma agents, they are also employed for treatment of such diseases as edema, hydropsy, altitude sickness, upper gut ulcer, chronic kidney deficiency, Parkinson's disease, and epilepsy seizures which are not affected by other drugs. It was noticed that CA inhibitors are effective in the treatment of certain cancers. For instance, it was identified that CA inhibitors suppress the growth of leukemia, melanoma, lung, ovarian, colon, kidney, prostate, breast, and CNS cancer cells (C. Supuran et al. (2000), Eur. J. Med. Chem. 35, 867-874). Namely, the carbonic anhydrases IX and XII are directly related to cancer development. The use of CA IX-specific inhibitor set for detection and treatment of pre-cancer and neoplastic state is described in (WO 2004/048544). It was established that CA inhibitors are useful diuretics for the treatment of patients which suffer from oedema and heart deficiency. It is supposed that inhibition of the CA II activity could be useful for the diminishment of the bone resorption. It was shown in prokaryotes that the carbonic anhydrases are essential for respiration, carbon dioxide transport and photosynthesis. Therefore it was hypothesized that carbonic anhydrase inhibitors could be used as antibiotics. Ethoxzolamide was even used for the treatment of meningitis. It was noticed that carbonic anhydrase inhibitors possess an antimallarial activity. (Merlin, C. Master, M. et al. (2003), J. Bacteriol. 185(21), 6415-24; Pastorekova, S. Parkkila, S. et al. (2004), J. Enzyme Inhib. Med. Chem. 19(3), 199-229; WO 2005/107470).

Significant consideration in the patent literature is given to various heterocyclic derivatives of sulfonamides which are carbonic anhydrase inhibitors and are the basis for pharmaceutical agents. For example, various thiophene derivatives are widely analyzed. Thienothiopyrane derivatives as thiophene derivatives condensed to the other rings are described in (US 7030250, US 5157129, US 5120757, US 5091409 and like). One of the thienothiopyrane derivatives is known as the pharmaceutical agent dorzolamide, which is used alone or in combination with other compounds for glaucoma treatment (US 6316443, 6248735, LT 3368, and like). Thienothiazinesulfonamide is the other class of thiophene sulfonamide derivatives (for instance US 5646142, US 5424448, US 5093332, US 5538966, and like). One of such derivatives, namely brinzolamide is an antiglaucoma agent. Other noted condensed derivatives of thiophene sulfonamides are thienothiadiazine derivatives (US 5510347, US 5464831), thienothiophene derivatives (US 4929549, US 4894390 and like), benzenethiophene derivatives (US 4788192, US 4668697), thienofurane derivatives (US 4798831), thienopyridine derivatives (US 4731368), thienopyrrole derivatives (US 4751231). A number of thiophene sulfonamide derivatives, non-condensed to other rings, were also patented, especially before 1990 (US 5378703, US 5240923, US 4847289, US 4929637 and like). Besides the thiophene derivatives a number of thiazole sulfonamides (for instance US 5519040) were patented. Primarily the benzothiazolesulfonamide derivatives were described (US 5059613, US 4975447 and like). One such compound, ethoxzolamide is antiglaucomal drug. Sulfonamides of thiadiazole class are still widely analyzed in patent literature (US 2004/0146955, US 5242937, US 5225424, US 5055480, US 5010204 and like). Two of thiadiazolesulfonamides are used as pharmaceutical agents - acetazolamide and methazolamide. Other classes of compounds are not analyzed in such a detail.

Various substituted non-condensed benzenesulfonamides are well-known and widely analyzed as CA inhibitors, although not much data is patented (US 2004/0146955; Mincione, F. Starnotti, M. et al. (2005), Biorg. Med. Chem. Lett. 15, 3821-3827; Poulsen, S.-A. Bornaghi, L. F. Healy, P. C. (2005), Biorg. Med. Chem. Lett. 15, 5429-33; US 4687855). Several pharmaceutical agents, for example, dichlorophenamide and indisulam, were based on the above mentioned compounds. Indisulam is presently being tested as an anticancer agent in phase II clinical trials.

During the last several years significant attention was shifted towards benzenesulfonamides which are inhibitors of CA and COX-2 (cyclooxygenase 2) (for instance US 2005/0222251, WO 2004/014352, WO 03/013655 and like). COX-2 inhibitors such as celecoxib, valdecoxib, and deracoxib are used as active materials for pharmaceutical agents. These preparations belong to the class of antiphlogistic non-steroid drugs used for arthritis treatment and painkilling. The antiphlogistic effect of Celecoxib-type substituted pyrazolylbenzenesulfonamides is widely analyzed (WO 95/15316).

Sulfonamide derivatives of heterocyclic system benzimidazo[1,2*-c*][1,2,3]thiadiazole are sulfonamides of the new class where sulfonamide group is attached to the benzene ring of the condensed three-ring system. As far as known to the authors of this invention, there are no mention in the literature about any three-ring condensed heterocyclic system with sulfonamide group attached to the benzene ring of this condensed system and such compound activity towards CA inhibition.

There are descriptions in the literature of other three-ring condensed heterocyclic systems with sulfonamide group attached to the heterocyclic ring and such compound possession of CA inhibitory activity (US 5681834, US 5334591, US 5308842, US 5235059, US 5175284 and like).

Despite the fact that a large number of different sulfonamides have been synthesized to date, the available pharmaceutical agents created on the basis of these sulfonamides have a number of shortcomings. One of the main shortcomings is the non-selective inhibition of all carbonic anhydrases throughout the whole body. This results in various unexpected side effects, mostly because of non-specific inhibition of all CA isoforms and their toxicity.

Presently clinically used CA inhibitors, when acting non-specifically, cause a number of side-effects. Especially toxic are systemic inhibitors. They cause electrolyte disbalance, drowsiness, head-ache, depression, apathy, malaise, irritability, nervousness, fatigue, gut irritability, anorexia, nausea, thirst, obstruction, muscle weakness, tremor, hyper- and hypoglycaemia, kidney pain, disuria, bone marrow depression, metabolic acidosis and other.

Therefore, the creation of isoform-specific or organ-selective sulfonamide inhibitors is still an important task.

### Summary of the Invention

This invention describes new sulfonamides with general structural formula (I) 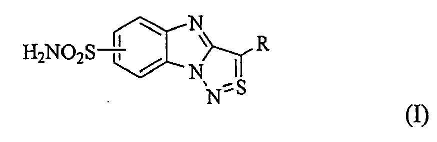 where R is H, Cl, SCH₃, SO₂CH₃, morpholine, thiophenyl, N(CH₃)₂, piperidine, N-methylpiperazine, pyrrolidine, and the sulfonamide group H₂NO₂S- is in 7 position or the 5, 6, or 8 position.

The objects of the invention are also the non-toxic, pharmaceutically acceptable salts of the sulfonamides of general formula (I). They include all salts which retain activity comparable to original compounds and do not attain any harmful and undesirable effects. Such salts are obtained from compounds with general structural formula (I) and basic nitrogen, by mixing their solution with pharmacologically acceptable non-toxic organic and inorganic acids, such as hydrogen chloride, butane diacid, citric acid, tartaric acid, phosphoric acid, sulphuric acid and other.

The examples of the implementation of the invention are compounds with sulfonamide group (H₂NO₂S-) in the 7^{th} position.

Examples of the invented compouds are selected compounds from the group comprising:
3-chlorobenzimidazo[1,2-*c*][1,2,3]thiadiazol-7-sulfonamide;
3-morpholinbenzimidazo[1,2-*c*][1,2,3]thiadiazol-7-sulfonamide;
3-phenylthiobenzimidazo[1,2-*c*][1,2,3]thiadiazol-7-sulfonamide;
3-methylthiobenzimidazo[1,2-*c*][1,2,3]thiadiazol-7-sulfonamide;
benzimidazo[1,2-*c*][1,2,3]thiadiazol-7-sulfonamide;
3-methylsulfonylbenzimidazo[1,2-*c*][1,2,3]thiadiazol-7-sulfonamide,
where all above listed compounds exhibit CA inhibitor properties.

The new intermediate compounds described below which could be used for the synthesis of sulfonamides with general formula (I) are also the subject of the invention.

### Detailed Description of the Invention

New compounds of the invention can be obtained according to general synthesis schemes A-G:
A) the scheme of 3-chlorobenzimidazo[1,2-*c*][1,2,3]thiadiazol-7-sulfonamide (compound 3) synthesis. 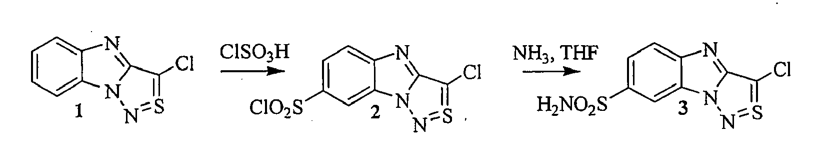
   Synthesis of the starting material (compound **1**) is described in: Tumkevicius, S. Labanauskas, L. Bucinskaite, V. Brukstus, A. Urbelis, G. (2003), Tetrahedron Lett. 44, 6635-38. Electrophylic substitution at the thiadiazole **1** goes to the 7^{th} position; therefore using chlorsulfonic acid the sulfonyl chloride **2** is obtained. Chlorine atom of the sulfonyl chloride group of the compound **2** is easily exchanged with the amino group by applying aqueous ammonia in tetrahydofurane, obtaining the compound **3** with general structural formula (I).
B) the scheme of 3-morpholin- and 3-phenylthiobenzimidazo[1,2-*c*][1,2,3]thiadiazol-7-sulfonamide (compounds **4, 5**) synthesis. 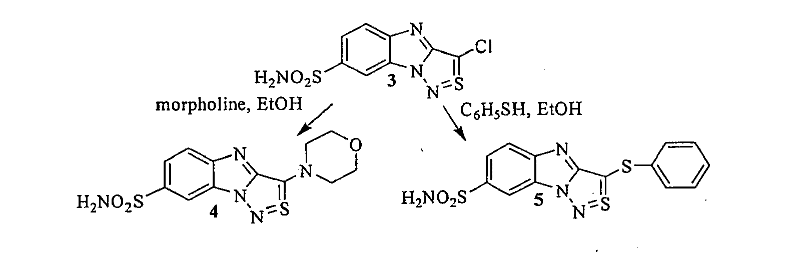
   Sulfonamides **4** and **5** are obtained from compound **3** using morpholine and thiophenol in ethanol. The compounds with general formula (I) and substituents R - N(CH₃)₂, piperidine, N-methylpiperazine, pyrrolidine groups could be synthesyzed in the same way.
C) the scheme of 3-methylthiobenzimidazo[1,2-*c*][1,2,3]thiadiazol-7-sulfonamide (compound **9**) synthesis. 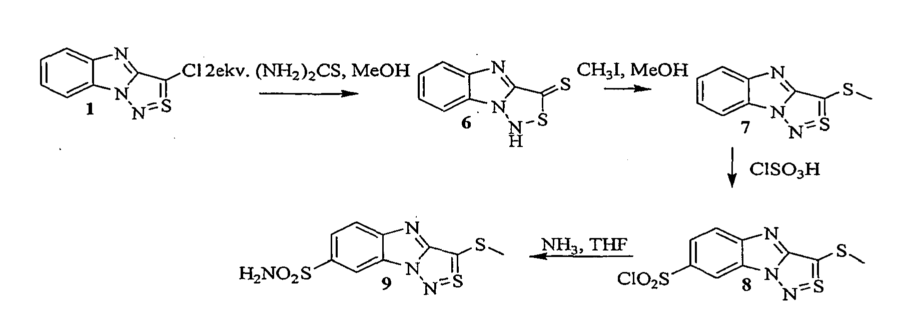
   Thiadiazole is first treated with thiourea leading to thione **6**. A different method for synthesis of the thione **6** is described in: Tumkevicius, S. Labanauskas, L. Bucinskaite, V. Brukstus, A. Urbelis, G. (2003), Tetrahedron Lett. 44, 6635-38. However, obtaining the compound **6** through thiourea is not described in the above mentioned manuscript. The obtained thione **6** reacts with methyl iodide producing methylated derivative **7**. Reaction of thiadiazole **7** with chlorosulfonic acid yields sulfonyl chloride **8**. Chlorine atom of the sulfonyl chloride group of the compound **8** can be readily exchanged by amino group using aqueous ammonia in tetrahydrofurane. The resultant sulfonamide **9** satisfies the general formula (I).
D) the scheme of benzimidazo[1,2*-c*][1,2,3]thiadiazol-7-sulfonamide (compound **12**) synthesis. 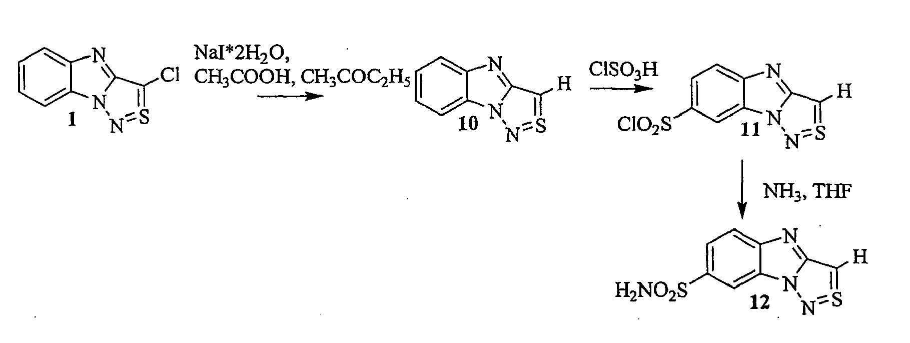
   Thiadiazole **1** is treated with aqueous sodium iodide and acetic acid in 2-butanone. The result of this reaction is thiadiazole **10** which reacts with chlorsulfonic acid yielding sulfonyl chloride **11.** Chlorine atom of the sulfonyl chloride group of compound **11** is readily exchanged by amino group using aqueous ammonia in tetrahydrofurane. This reaction results in compound **12** corresponding to general formula (I).
E) the scheme of 3-methylsulfonylbenzimidazo[1,2-*c*][1,2,3]thiadiazol-7-sulfonamide (compound **15**) synthesis. 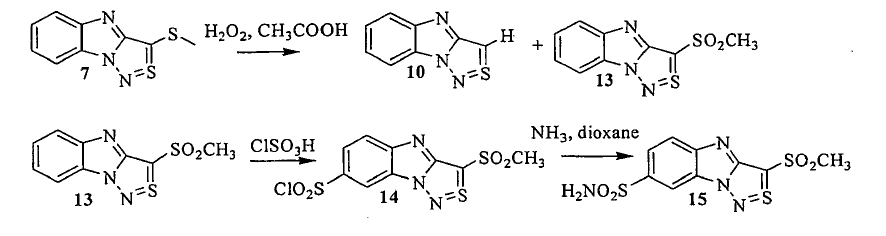
   Methyl sulfonyl derivative **13** is prepared by oxidation of **7**. It should be mentioned that oxidation of 7 with hydrogen peroxide in acetic acid yields a mixture of compounds **10** and **13**. The mixture of compounds **10** and **13** is separated by flash chromatography. Thiadiazole **13** reacts with chlorsulfonic acid yielding sulfonyl chloride **14**. Chlorine atom of the sulfonyl chloride group of the compound **14** is readily exchanged with amino group using aqueous ammonia in dioxane and the resultant sulfonamide **15** corresponds to general formula (I).
F) the scheme of 3-dimethylaminobenzimidazo[1,2-*c*][1,2,3]thiadiazol-6-sulfonamide (compound **22**) synthesis.
   Synthesis of (5(6)-bromo-1*H*-benzimidazol-2-yl)-methanol (**16 a, b**) is described in Khan, M. K. Mohammady, A. Fauzia, A. Y. (1972), J. Sci. and Ind. Res. 15, 11-12. Amination of compound (**16 a, b**) with hydroxylamino-O-sulfonic acid yields a mixture of 1-aminobenzimidazoles **17** and **18**, which are inseparable by column chromatography or fractional crystallization. Therefore, they are employed in the next reaction with thionyl chloride expecting that the cyclic products will have different physical properties. 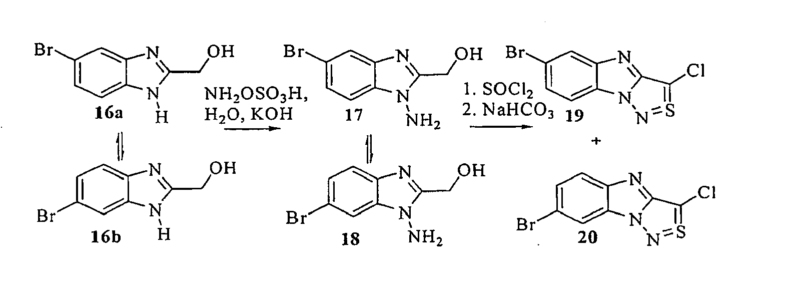 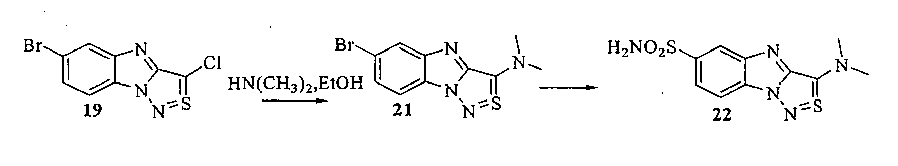
   Indeed, compounds **17** and **18** reacts with thionyl chloride to give a mixture of 6-bromo-and 7-bromobenzimidazothiadiazoles **19, 20**. Isomers **19, 20** are separated by flash chromatography. Compound **19** undergoes chlorine substitution reaction with dimethylamine to form the compound **21.** Thiadiazole **22** can be synthesized from compound **21** in accordance with techniques where the preparation of aromatic sulfonyl chlorides and sulfonamides from their halides is described (Pandya, R. Murashima, T. Tedeschi, L. Barrett, A. G. M. (2003), J. Org. Chem. 68, 8274-8276; Graham, S. L. Scholz, T.H. (1986), Synthesis, 1031-1032; Hamada, T. Yonemitsu, O. (1986), Synthesis, 852-854).
G) the scheme of 3-dimethylaminobenzimidazo[1,2*-c*][1,2,3]thiadiazol-5- and 8-sulfonamides (compounds **30**, **32**) synthesis.
   Thiadiazoles bearing sulfonamide group in 5 and 8 positions of the ring system can be synthesized in a similar way to compound **22**. The synthesis of a starting material **23** is described in: Sunder, S. Peet, N.P. (1979), J.Heterocycl. Chem., 16, 33-37. Compound 23 can undergo cyclization reaction with glycolic acid to form 4(7)-bromo-1*H*-benzimidazol-2-yl) methanol (**24 a, b**). Cyclization reaction can occur under the same conditions as used for the synthesis of 4(7)-bromo-2-methyl-1*H*-benzimidazole (Dandegaonker, Recanker, (1961), J. Karnatak Univ. 6, 25, 29, 30).

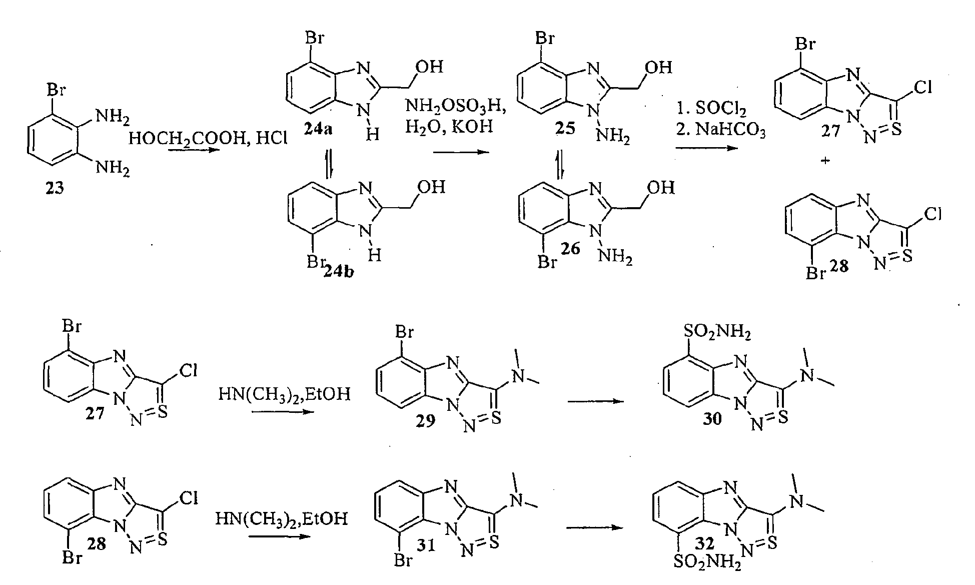

Following steps in the synthesis of compounds **30** and **32** are performed in the same way as for the compound **22**.

### Brief Description of the Drawings

To illustrate the main characteristics of the new compounds this description contains:
Fig. 1. Typical isothermal titration calorimetry data of bovine carbonic anhydrase binding to: A) pentafluorbenzensulfonamide, B) compound of the invention.
Fig. 2. Stabilization of bovine carbonic anhydrase II (b-CAII) by compound of the invention as determined by the fluorescence-based thermal shift assay.
Fig. 3. Bovine carbonic anhydrase II melting temperature (Tm) dependence on the concentration of an invented compound (Lt), determined by the thermal shift assay.

### Embodiments of the Invention

Represented below are specific examples of invention compounds, synthesis, including intermediate compounds required for objective compound synthesis (when there is no data of such synthesis in the literature). These examples are presented only for illustrative purpose of the invention; they do not limit the scope of the invention.

### Example 1. Production of intermediate compound 3-chlorobenzimidazo[1,2-c][1,2,3]thiadiazol-7-sulfonyl chloride (compound 2).

The 3-chlorobenzimidazo[1,2*-c*][1,2,3]thiadiazole (compound 1) (0.3 g, 1.43 mmol), prepared according to known methodology, is added slowly with stirring to CISO₃H (3 mL) at - 5°C. The reaction mixture is allowed to warm to room temperature and remains under stirring for 24 h. The excess of acid is then hydrolyzed with ice. The product is extracted with chloroform. The combined chloroform layers are washed with H₂O, dried over anhydrous Na₂SO₄, yielding chlorosulfonyl compound after evaporation. The resultant compound is crystalline and has a bright yellow color.
Yield: 0.38 g (86%), mp 184-185°C.
¹H NMR (300 MHz, CDCl₃) δ ppm 8.1 (1H, d, J = 9 Hz, CH(5)), 8.23 (1H, dd, J = 2 and 9 Hz, CH(6)), 8.94 (1H, d, J = 2 Hz, CH(8)).
The crude material is used for next step without further purification.

### Example 2. Preparation of 3-chlorobenzimidazo[1,2-c][1,2,3]thiadiazol-7-sulfonamide (compound 3).

To a solution of 3-chlorobenzimidazo[1,2-*c*][1,2,3]thiadiazol-7-sulfonyl chloride (compound 2) (0.04 g, 0.13 mmol) in tetrahydrofurane (2 mL) is added dropwise with stirring an aqueous NH₃ solution (0.1 mL, 25%). After addition the reaction mixture is stirred at ambient temperature for 15 min. The solid that precipitates is filtered, washed (NaHCO₃/H₂O, H₂O). Recrystallization is accomplished from acetic acid. The resultant compound is crystalline and has orange color.
Yield: 0.03 g (80%), mp 242-243°C.
¹H NMR (300 MHz, DMSO-d₆) δ ppm 7.49 (2H, s, NH₂), 8.01 (2H, s, CH(5), CH(6)), 8.62 (1H, s, CH(8)).
¹³C NMR (75 MHz, DMSO-d₆) δ ppm 112.00, 121.59, 125.77, 127.34, 127.37, 136.18, 154.13, 154.83.
MS (+ESI) (m/z, %): (M+H)⁺ 289, 100%, (M+H)⁺ 291, 50%.
Analysis (C₈H₅ClN₄O₂S₂): calcd: C 33.28%, H 1.75%, N 19.40%; found: C 33.39%, H 1.8%, N 19.63%.

### Example 3. Preparation of 3-morpholinbenzimidazo[1,2-c][1,2,3]thiadiazol-7-sulfonamide (compound 4).

The mixture of 3-chlorobenzimidazo[1,2*-c*][1,2,3]thiadiazol-7-sulfonamide (compound 3) (0.02 g, 0.069 mmol), morpholine (0.012 g, 0.14 mmol), and ethanol (20 mL) is refluxed for 1.5 h. The reaction mixture is cooled to room temperature and the precipitate is filtered, washed (cold H₂O). Recrystallization is accomplished from acetic acid.
Yield: 0.02 g (85%), mp 253-254°C.
¹H NMR (300 MHz, DMSO-d₆) δ ppm 3.9 (8H, s, (CH₂)₄), 7.41 (2H, s, NH₂), 7.88 (2H, s, CH(5), CH(6)), 8.44 (1H, s, CH(8)).
¹³C NMR (75 MHz, DMSO-d₆) δ ppm 50.38, 65.79, 111.6, 121.27, 124.00, 126.4, 135.15, 146.94, 151.7, 154.23.
Analysis (C₁₂H₁₄N₅O₃S₂): calcd C 42.47%, H 3.86%, N 20.63%; found: C 42.56%, H 3.94%, N 20.47%.

### Example 4. Production of 3-phenylthiobenzimidazo[1,2-c][1,2,3]thiadiazol-7-sulfonamide (compound 5).

The mixture of 3-chlorobenzimidazo[1,2*-c*][1,2,3]thiadiazol-7-sulfonamide (compound 3) (0.1 g, 0.35 mmol), thiophenol (0.039 g, 0.35 mmol), and ethanol (50 mL) is refluxed for 1.5 h. The reaction mixture is cooled to room temperature, ethanol is evaporated and the resultant precipitate is filtered, washed (NaHCO₃/H₂O, H₂O). Recrystallization is accomplished from acetic acid. The resultant compound is crystalline and has orange color.
Yield: 0.02g (85%), mp 225-226°C.
¹H NMR (300 MHz, DMSO-d₆) δ ppm 7.3-7.6 (5H, m, SC₆H₅), 7.65 (2H, s, NH₂), 7.96 (2H, s, CH(5) and CH(6)), 8.59 (1H, s, CH(8)).
¹³C NMR (75 MHz, DMSO-d₆) δ ppm 112, 121.54, 125.43, 127.13, 129.9, 130.9, 131.39, 131.97, 132.51, 136.15, 154.76, 155.7.
Analysis (C₁₄H₁₀N₄O₂S₃): calcd C 46.39%, H 2.78%, N 15.46%; found: C 46.26%, H 2.87%, N 15.37%.

### Example 5. Production of intermediate compound benzimidazo[1,2-c][1,2,3]thiadiazol-3-thione (compound 6).

The mixture of 3-chlorobenzimidazo[1,2*-c*][1,2,3]thiadiazole (compound **1**) prepared according to known methodics (0.5 g, 2.38 mmol), thiourea (0.4 g, 5.26 mmol), and ethanol (20 mL) is refluxed for 0.5 h. The reaction mixture is cooled to room temperature, the precipitate is filtered, washed (cold methanol); dissolved in 0.2 M NaOH. The solution is filtered and acidified with acetic acid to pH ∼5. The resultant precipitate is filtered and recrystallization is accomplished from dioxane. The resultant compound is crystalline and has bright orange color.
Yield: 0.34g (69%), mp 236-237°C.

### Example 6. Production of intermediate compound 3-methylthiobenzimidazo[1,2-c][1,2,3]thiadiazole (compound 7).

The mixture of benzimidazo[1,2*-c*][1,2,3]thiadiazol-3-thione (compound **6**) (0.2 g, 0.96 mmol), methyl iodide (0.2 g, 1.4 mmol), and methanol (40 mL) is refluxed for 0.5 h, solvent is evaporated, the resultant precipitate is filtered, washed (NaHCO₃/H₂O, H₂O). Recrystallization is accomplished from H₂O/ethanol (2:1) mixture. The resultant compound is crystalline and has orange color.
Yield: 0.18g (86%), mp 120-121°C.
¹H NMR (300 MHz, DMSO-d₆/CCl₄) δ ppm 2.97 (3H, s, SCH₃), 7.26 (1H, t, J= 8Hz, CH(7)), 7.51 (1H, t, J= 8Hz, CH(6)), 7.77 (1H, d, J= 8Hz, CH(5)), 8.09 (1H, d, J= 8Hz, CH(8)).
¹³C NMR (75 MHz, DMSO-d₆/CCl₄) δ ppm 18.49, 113.25, 120.33, 121.18, 127.66, 128.21, 134.79, 153.04, 153.63.
Analysis (C₉H₇N₃S₂): calcd: C 48.85%, H 3.19%, N 18.99%; found: C 48.93%, H 2.96%, N 18.89%.

### Example 7. Production of intermediate compound 3-methylthiobenzimidazo[1,2-c][1,2,3]thiadiazol-7-sulfonyl chloride (compound 8).

The 3-methylthiobenzimidazo[1,2-*c*][1,2,3]thiadiazole (compound 7) (0.1 g, 0.45 mmol) is added slowly with stirring to ClSO₃H (1 mL) at -5°C. The reaction mixture is allowed to warm to room temperature and remains under stirring for 24 h. The excess of acid is then hydrolyzed with ice. The product is extracted with chloroform. The combined chloroform layers are washed with H₂O, dried over anhydrous Na₂SO₄. Chloroform is evaporated. The resultant compound is crystalline and has a bright yellow color.
Yield: 0.12g (86%), decomposition at 190°C.
¹H NMR (300 MHz, CDCl₃) δ ppm 2.99 (1H, s, SCH₃), 8.05 (1H, dd, J= 0.6 and 9Hz, CH(5)), 8.17 (1H, dd, J= 2 and 9 Hz, CH(6)), 8.90 (1H, dd, J= 0.6 and 2Hz, CH(8)).
The crude material is used for next step without further purification.

### Example 8. Production of 3-methylthiobenzimidazo[1,2-c][1,2,3]thiadiazol-7-sulfonamide (compound 9).

To a solution of 3-methylthiobenzimidazo[1,2*-c*][1,2,3]thiadiazol-7-sulfonyl chloride (compound **8**) (0.04 g, 0.125 mmol) in tetrahydrofurane (2 mL) is added dropwise with stirring an aqueous NH₃ solution (0.1 mL, 25%). After addition, the reaction mixture is stirred at ambient temperature for 15 min. The solid that precipitated is filtered, washed (NaHCO₃/H₂O, H₂O). Recrystallization is accomplished from acetic acid. The resultant compound is crystalline and has an orange color.
Yield: 0.02g (53%), mp 260-261°C.
¹H NMR (300 MHz, DMSO-d₆) δ ppm 2.99 (1H, s, SCH₃), 7.47 (2H, s, NH₂), 8.00 (2H, s, CH(5) and CH(6)), 8.58 (1H, s, CH(8)).
¹³C NMR (75 MHz, DMSO-d₆) δ ppm 18.58, 112.00, 121.47, 125.04, 126.69, 135.95, 138.72, 154.00, 154.98.
Analysis (C₉H₈N₄O₂S₃): calcd: C 35.99%, H 2.68%, N 18.65%; found: C 36.16%, H 2.62%, N 18.80%.

### Example 9. Production of intermediate compound benzimidazo[1,2-c][1,2,3]thiadiazole (compound 10).

The mixture of 3-chlorobenzimidazo[1,2*-c*][1,2,3]thiadiazole (compound **1**) prepared according to known methodics (0.2 g, 0.95 mmol), NaI·2H₂O (0.9 g, 4.7 mmol), acetic acid (10 mL) and 2-butanone (50 mL) is refluxed for 5 h. The solvent is evaporated, the resultant mass is mixed with aqueous Na₂S₂O₃ solution. Recrystallization is accomplished from H₂O/ethanol (10:1) mixture. The resultant compound is crystalline and has orange color.
Yield: 0.06g (38%), mp 158-160°C.
¹H NMR (300 MHz, CDCl₃) δ ppm 7.34 (1H, t, J= 8Hz, CH(7)), 7.6 (1H, t, J= 8Hz, CH(6)), 7.94 (1H, d, J= 8Hz, CH(5)), 8.2 (1H, d, J= 8Hz, CH(8)), 8.47 (1H, s, CH(3)).
¹³C NMR (75 MHz, CDCl₃) δ ppm 113.29, 116.76, 120.47, 121.15, 127.71, 128.28, 154.98, 155.38.
MS (EI) (m/z, %): M⁺ 175, 93%.
Analysis (C₈H₅N₃S): calcd: C 54.84%, H 2.88%, N 23.98%; found: C 54.65%, H 2.37%, N 23.60%.

### Example 10. Production of intermediate compound benzimidazo[1,2-c][1,2,3]thiadiazol-7-sulfonyl chloride (compound 11).

The benzimidazo[1,2*-c*][1,2,3]thiadiazole (compound **10**) (0.06 g, 0.34 mmol) is added slowly with stirring to ClSO₃H (0.6 mL) at -5°C. The reaction mixture is allowed to warm to room temperature and remains under stirring for 24 h. The excess of acid is then hydrolyzed with ice. The product is extracted with chloroform. The combined chloroform layers are washed with H₂O, dried over anhydrous Na₂SO₄. Chloroform is evaporated. The resultant compound is crystalline and has a bright yellow color.
Yield: 0.06g (64%), mp 188-189°C.
¹H NMR (300 MHz, CDCl₃) δ ppm 8.1 (1H, dd, J= 0.6 and 9Hz, CH(5)), 8.24 (1H, dd, J= 2 and 9 Hz, CH(6)), 8,74 (1H, s, CH(3)), δ (1H, dd, J= 0.6 and 2Hz, CH(8)).

The crude material is used for next step without further purification.

### Example 11. Production of benzimidazo[1,2-c][1,2,3]thiadiazol-7-sulfonamide (compound 12).

To the solution of benzimidazo[1,2*-c*][1,2,3]thiadiazol-7-sulfonyl chloride (compound **11**) (0.06 g, 0.22 mmol) in tetrahydrofurane (2 mL) is added dropwise with stirring an aqueous NH₃ solution (0.15 mL 25%). After addition, the reaction mixture is stirred at ambient temperature for 15 min. The solid that precipitated is filtered, washed (NaHCO₃/H₂O, H₂O). Recrystallization is accomplished from acetic acid. The resultant compound is crystalline and has a yellow color.
Yield: 0.039g (70%), mp 245-246°C.
¹H NMR (300 MHz, DMSO-d₆): δ ppm 7.46 (2H, s, NH₂), 7.98 (2H, s, CH(5) and CH(6)), 8.63 (1H, s, CH(8)), 9.3 (1H, s, CH(3)).
¹³C NMR (75 MHz, DMSO-d₆): δ ppm 112.08, 121.22, 122.69, 125.18, 125.94, 135.37, 155.3, 157.75.
Analysis (C₈H₆N₄O₂S₂): calcd: C 37.79 %, H 2.38%, N 22.03 %; found: C 37.86 %, H 2.41 %, N 22.18 %.

### Example 12. Production of intermediate compound 3-methylsulfonylbenzimidazo[1,2-c][1,2,3]thiadiazole (compound 13).

To a solution of 3-methylthiobenzimidazo[1,2-*c*][1,2,3]thiadiazole (compound 7) (0.3 g, 1.36 mmol) in acetic acid (7 mL) is poured H₂O₂ (0.65 g, 35%). The reaction mixture is kept at room temperature for five days. The reaction solvent is evaporated and residue is poured into NaHCO₃/H₂O solution and mixed up carefully. The precipitate contains a mixture of compounds **10** and **13.** The compounds are separated by flash chromatography (ethylacetate). The resultant compound **13** is crystalline and has a dark red color.
Compound **10**: yield: 0.03g (1%), mp 158-160°C.
R_{f}= 0.13 (ethylacetate).
3-methylsulfonylbenzimidazo[1,2-c][1,2,3]thiadiazole (compound **13**): yield: 0.19g (56%), mp 186-187°C.
R_{f}= 0.73 (ethylacetate).
¹H NMR (300 MHz, CDCl₃) δ ppm 3.64 (3H, s, SO₂CH₃), 7.47 (1H, t, J= 8Hz, 7-H), 7.71 (1H, t, J= 8Hz, CH(6)), 8.03 (1H, d, J= 8Hz, CH(5)), 8.24 (1H, d, J= 8Hz, CH(8)).
¹³C NMR (75 MHz, CDCl₃) δ ppm 44.82, 113.39, 121.68, 122.2, 128.35, 129.86, 141.86, 149.59, 155.6.
Analysis (C₉H₇N₃O₂S₂): calcd: C 42.67%, H 2.79%, N 16.59%; found: C 42.86%, H 2.75%, N 16.48%.

### Example 13. Production of intermediate compound 3-methylsulfonylbenzimidazo[1,2-c][1,2,3]thiadiazol-7-sulfonyl chloride (compound 14).

The 3-methylsulfonylbenzimidazo[1,2-*c*][1,2,3]thiadiazole (compound **13**) (0.05 g, 0.2 mmol) is added slowly with stirring to ClSO₃H (0.6 mL) at -5°C. The reaction mixture is allowed to warm to room temperature and remains under stirring for 24 h. The excess of acid is then hydrolyzed with ice. The solid that precipitated is filtered. The resultant compound is crystalline and has orange color.
Yield: 0.05g (72%), decomposition at 210°C.
¹H NMR (300 MHz, DMSO-d₆) δ ppm 3.73 (3H, s, SO₂CH₃), 8.1 (1H, d, J = 9 Hz, CH(5)), 8 (1H, dd, J = 2 and 9 Hz, CH(6)), 8.42 (1H, s, CH(8)).

The crude material was used for next step without further purification.

### Example 14. Production of 3-methylsulfonylbenzimidazo[1,2-c][1,2,3]thiadiazol-7-sulfonamide (compound 15).

To a solution of 3-methylsulfonylbenzimidazo[1,2-*c*][1,2,3]thiadiazol-7-sulfonyl chloride (compound **14**) (0.032 g, 0.09 mmol) in dioxane (20mL) is added dropwise with stirring an aqueous NH₃ solution (0.1 mL, 25%). After addition the reaction mixture is stirred at ambient temperature for 0.5 h. The reaction solvent is evaporated and residue is washed (NaHCO₃/H₂O, H₂O). Recrystallization is accomplished from acetic acid. The resultant compound is crystalline and has orange color.
Yield: 0.018 g (60%), mp 249-250°C.
¹H NMR (300 MHz, DMSO-d₆): δ ppm 3.69 (3H, s, SO₂CH₃), 7.56 (2H, s, NH₂), 8.1 (2H, s, CH(5), CH(6)), 8.7 (1H, s, CH(8)).
¹³C NMR (75 MHz, DMSO-d₆): δ ppm 44.96, 112.13, 121.7, 126.44, 126.95, 132.96, 137.24, 152.79, 155.73.
Analysis (C₉H₈N₄O₄S₃): calcd: C 32.52%, H 2.43%, N 16.86%; found: C 32.61%, H 2.44%, N 16.75%.

### Example 15. Production of intermediate compounds (1-amino-5-bromo-1H-benzimidazol-2-yl)methanol (compound 17) and (1-amino-6-bromo-1H-benzimidazol-2-yl)methanol (compound 18).

The 5(6)-bromo-1H-benzimidazol-2-methanol (**16 a, b**) (5.2 g, 22.7 mmol), prepared to known methodology, is dissolved in a solution of KOH (4.8 g, 72.8 mmol) in H₂O (40 mL). To the mixture is added under stirring at 40°C solution of NH₂0SO₃H (6g, 50mmol) in H₂O (15 mL), neutralized NaHCO₃. Upon the completion of the exothermal reaction the mixture is still incubated at 40-50°C for 0.5 h and then cooled to room temperature. The resultant precipitate is filtered and recrystallization is accomplished from water. The mixture of compounds **17** and **18** is obtained in 1:1 ratio (according to ¹H NMR). The mixture of these compounds is crystalline, white.
Overall yield: 3.7g (67%).
¹H NMR (300 MHz, DMSO-d₆): δ ppm 4.73 (4H, s, 2CH₂), 5.43 (1H, s, OH), 5.45 (1H, s, OH), 6.01 (2H, s, NH₂), 6.03 (2H, s, NH₂), 7.31 (1H, d, J= 9Hz, ArH), 7.39 (1H, d, J= 9Hz, ArH), 7.46 (1H, d, J= 9Hz, ArH), 7.53 (1H, d, J= 9Hz, ArH), 7.67 (1H, s, ArH), 7.73 (1H, s, ArH).
¹³C NMR (75 MHz, DMSO-d₆): δ ppm 55.81, 55.81, 112.48, 113.46, 114.15, 114.98, 121.46, 122.01, 124.93, 125.36, 135.73, 137.78, 139.62, 141.88, 156.17, 156.48.
IR (v, cm⁻¹): 3350, 3313, 3184, 3120 NH₂.
Analysis (C₈H₈BrN₃O): calcd: C 39.67%, H 3.31 %, N 17.36%; found: C 39.88%, H 3.52%, N17.46%.

### Example 16. Production of intermediate compounds 3-chloro-6-bromobenzimidazo[1,2-c][1,2,3]thiadiazole (compound 19) and 3-chloro-7-bromobenzimidazo[1,2-c][1,2,3]thiadiazole (compound 20).

The mixture of (1-amino-5-bromo-1H-benzimidazol-2-yl)-methanol (compound **17)** and (1-amino-6-bromo-1H-benzimidazol-2-yl)-methanol (compound **18)** (0.5 g, 2.1 mmol) is refluxed with SOCl₂ (5 mL) for 0.5 h. The excess of SOCl₂ is evaporated. The residue is washed (NaHCO₃/H₂O, H₂O). The mixture of products consists of compounds **19** and **20** in proportion 1:1. The compounds were separated by flash chromatography (dichlormethane: ethylacetate=10:1). Overall yield: 0.36 g (60%) (recrystallization is accomplished from methanol).

**20**: Yield: 0.15g (25%), mp 193-194°C.
R_{f}= 0.48 (dichlormethane: ethylacetate=10:1).
¹H NMR (300 MHz, DMSO-d₆): 7.7 (1H, dd, J= 2 and 9Hz, CH(6)), 7.79 (1H, dd, J= 0.5Hz and 9Hz, CH(5)), 8.46 (1H, dd, J= 0.5 and 2Hz, CH(8)).
¹³C NMR (75 MHz, DMSO-d₆): 112.76, 116.24, 123.03, 126.61, 129.36, 131.66, 151.72, 152.49.
Analysis (C₈H₃BrClN₃S): calcd: C 33.30%, H 1.05 %, N 14.56%; found: C 33.56%, H 1.15%, N 14.63%.
3-chloro-6-bromobenzimidazo[1,2-*c*][1,2,3]thiadiazole (compound 19) yield: 0,15g (25%), mp 153-154°C.
R_{f}= 0.59 (dichlormethane: ethylacetate=10:1).
¹H NMR (300 MHz, DMSO-d₆): 7.45 (1H; dd, J= 2 and 9Hz, CH(7)), 8.07 (1H, d, J= 2Hz, CH(5)), 8.19 (1H, d, J= 9Hz, CH(8)).
¹³C NMR (75 MHz, DMSO-d₆): 115.36, 121.57, 123.38, 123.77, 127.76, 129.38, 152.77, 154.55.
Analysis (C₈H₃BrClN₃S): calc: C 33.30 %, H 1.05 %, N 14.56 %; found: C 33.01 %, H 1.33 %, N 14.36 %).

### Example 17. Production of intermediate compound 6-bromo-3-dimethylaminbenzimidazo[1,2-c][1,2,3]thiadiazole (compound 21).

Mixture of 3-chloro-6-bromobenzimidazo[1,2*-c*][1,2,3]thiadiazole (2 g, 6.9 mmol) (compound 19), aqueous dimethylamine (0.94 g, 33%), ethanol (100 mL) is refluxed for 3 h. Solvent is evaporated and the residue is washed with cold H₂O. Recrystallization is accomplished from methanol. The resultant compound is crystalline and has orange color.
Yield: 1.6 g (78%), mp 176-177 °C.
¹H NMR (300 MHz, CDCl₃): 3.49 (6H, s, N(CH₃)₂), 7.29 (1H, dd, J= 2 and 9Hz, CH(7)), 7.86 (1H, d, J= 9Hz, CH(8)), 7.98 (1H, d, J= 2Hz, CH(5)),
¹³C NMR (75 MHz, CDCl₃): 42.78, 113.79, 119.89, 122.84, 123.37, 127.23, 145.06, 151.7, 153.95.
Analysis (C₁₀H₉BrN₄S): calc: C 40.42 %, H 3.05 %, N 18.85 %; found: C 40.51 %, H 3.02 %, N 18.87%).

Inhibition of carbonic anhydrases (also all enzymes) is measured by determining the reduction in the speed of catalysis. Carbonic anhydrases are zinc-containing metalloenzymes, catalyzing the reversible reaction:

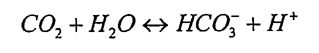

The inhibition of this reaction may be determined by measuring carbon dioxide consumption, bicarbonate appearance and the changes of pH (Krebs, J.F. and C.A. Fierke, (1993), J. Biol. Chem. 268(2), 948-54). Inhibitors bind to the active center of carbonic anhydrases, compete with the substrate and diminish the catalysed reaction. All sulfonamides bind to the active center and diminish this reaction. Inhibition is equivalent to binding. (Chakravarty, S. and K.K. Kannan, (1994), J. Mol. Biol. 243(2), 298-309; Lindskog, S. (1997), Pharmacol. Ther. 74(1), 1-20; Baird, T.T.Jr. et al. (1997), Biochemisny, 36(9), 2669-78). However, their binding and inhibitory efficiency varies greatly. Furthermore, the specificity of various sulfonamides, i.e., how much they inhibit particular isozymes, varies greatly. (Di Fiore, A. et al. (2005), Bioorg. Med. Chem. Lett. 15(7), 1937-42; Lee, D.A. and E.J. Higginbotham, (2005), Am. J. Health Syst. Pharm. 62(7), 691-9; Matulis, D. et al. Biochemistry, (2005), 44(13), 5258-66; Ozensoy, O. et al. (2005), Bioorg. Med. Chem. Lett. 15(21), 4862-6; Shank, R.P. et al. (2005), Epilepsy Res. 63(2-3), 103-12; Simone, G.D. et al. (2005), Bioorg. Med. Chem. Lett. 15(9), 2315-20). Sulfonamide binding to carbonic anhydrases is measured by a number of standard means. Most often used methods are isothermal titration calorimetry, surface plasmon resonance, and ultracentrifugation. (Myszka, D.G. et al. (2003), J. Biomol. Tech. 14(4), 247-69). Specificity is determined by measuring binding constants with various isozymes and also by measuring "true" binding constants (Matulis, D. and M.J. Todd, (2004), Trends in Biocalorimetry).

### Example 18. Determination of the specificity of CA binding and inhibition by isothermal titration calorimetry.

The heat evolved upon inhibitor (compound **3**, compound **4**, and compound **9**) binding to bovine carbonic anhydrase II and human carbonic anhydrase I was measured by isothermal titration calorimetry and compared to known carbonic anhydras inhibitors, namely, trifluoromethanesulfonamide (TFMSA), acetazolamide (AZM), etoxzolamide (EZA), and pentafluorobenzenesulfonamide (PFS).

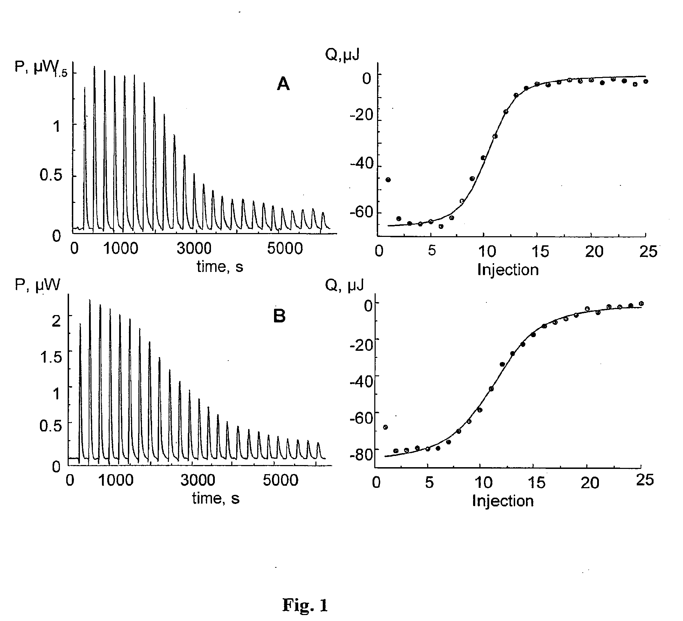

To illustrate the results, Figure 1 shows isothermal titration calorimetry curves of bovine carbonic anhydrase II binding to pentafluorobenzenesulfonamide (A) and 3-morpholinbenzimidazo[1,2*-c*][1,2,3]thiadiazol-7sulfonamide (**4**) (B).

The resultant "observed" binding constants were in the order of 10⁶-10⁷ M⁻¹ for all tested compounds of this invention. However, these constants depend on protonation events involving buffer, zinc, and inhibitor molecules. Therefore, the more precise measure is the calculated "true" or "intrinsic" binding constants that are not dependent on above mentioned protonation events. When the intrinsic binding constants were compared to the literature compounds, the advantage of the invented compounds was obvious. Below is the series of compounds in the order of decreasing their intrinsic binding constant. Numbers above the series show the times difference between the neighboring compounds. The range is due to different activities towards carbonic anhydrase II and I.

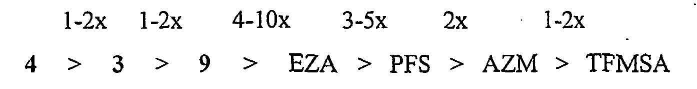

As seen from the series, the compounds **4, 3**, and **9** are all significantly better than literature compounds and could be potentially used as active ingredients of pharmaceutical agents.

### Example 19. Determination of the observed binding constants by the fluorescent thermal shift assay.

Inhibitor binding to carbonic anhytdrases was also measured by the fluorescent thermal shift assay, which employs 1-anilino-8-naphthalene sulfonate (ANS) probe that visualizes enzyme thermal stabilization by the inhibitor thus determining the binding constant.

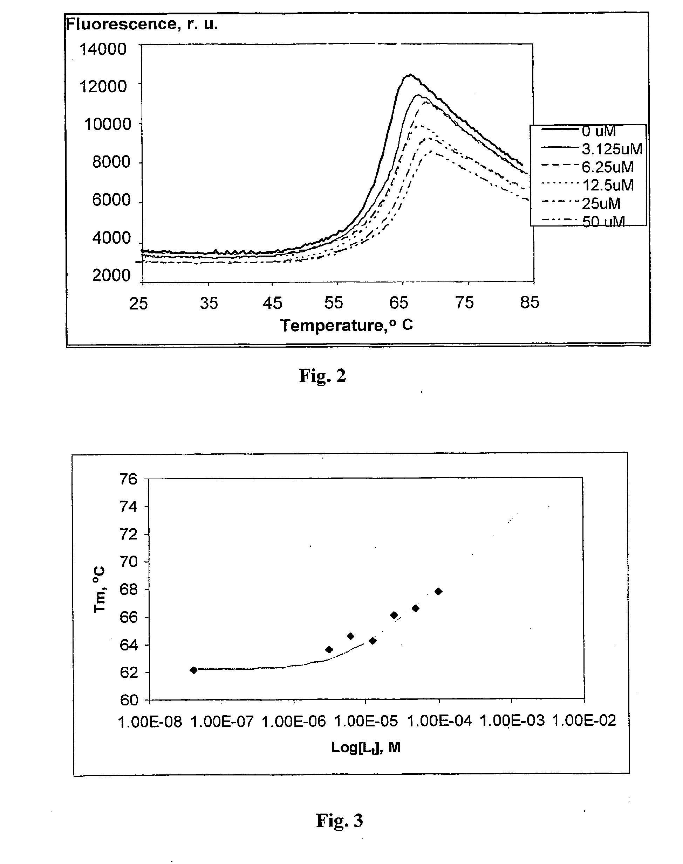

Figure 2 shows the enzyme raw unfolding curves and the increase in Tm upon increasing concentration of inhibitors. Figure 3 shows the Tm increase of bovine carbonic anhydrase II upon increasing concentration of the inhibitor, 3-morpholinbenzimidazo[1,2-c][1,2,3]thiadiazol-7-sulfonamide (4). The curve in Figure 3 shows the simulated curve according to literature model. Resultant data is consistent with the isothermal titration calorimetry data in determining the observed binding constants of the inhibitors.

Newly synthesized sulfonamides of general formula (I) 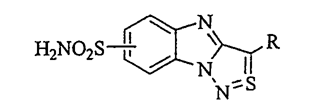 exhibit a comparable efficiency to the existing inhibitors used as drugs, but the invented compounds exhibit significantly better specificity than the existing compounds promising to solve the problem of non-specific binding of literature inhibitors.

## Claims

1. Benzimidazo[1,2*-c*][1,2,3]thiadiazole sulfonamides of general formula (I) 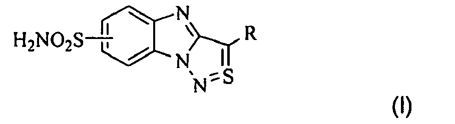 wherein R is H, Cl, SCH₃, SO₂CH₃, morpholine, tiophenyl, N(CH₃)₂, piperidine, N-methylpiperazine, and where sulfonamide group H₂NO₂S- is in 7 position or 5, 6, 8 position, and pharmaceutically acceptable salts thereof.

2. Benzimidazo[1,2-*c*][1,2,3]thiadiazole sulfonamides of general formula (I) according to claim 1, wherein R is H, Cl, SCH₃, SO₂CH₃, morpholine or tiophenyl and where sulfonamide group H₂NO₂S- is in 7 position, and pharmaceutically acceptable salts thereof.

3. Compound according to claim 2, selected from a group comprising of:
3-chlorobenzimidazo[1,2-*c*][1,2,3]thiadiazol-7-sulfonamide;
3-morpholinbenzimidazo[1,2-*c*][1,2,3]thiadiazol-7-sulfonamide;
3-phenylthiobenzimidazo[1,2-*c*][1,2,3]thiadiazol-7-sulfonamide;
3-methylthiobenzimidazo[1,2-*c*][1,2,3]thiadiazol-T-sulfonamide;
benzimidazo[1,2-c][1,2,3]thiadiazol-7-sulfonamide;
3-methylsulfonylbenzimidazo[1,2-*c*][1,2,3]thiadiazol-7-sulfonamide.

4. 3-chlorobenzimidazo[1,2-*c*][1,2,3]thiadiazol-7-sulfonyl chloride **2**.

5. 3-methylthiobenzimidazo[1,2-*c*][1,2,3]thiadiazole **7**.

6. 3-methylthiobenzimidazo[1,2-*c*][1,2,3]thiadiazol-7-sulfonyl chloride **8**.

7. Benzimidazo[1,2-*c*][1,2,3]thiadiazole **10**.

8. Benzimidazo[1,2-*c*][1,2,3]thiadiazol-7-sulfonyl chloride **11.**

9. 3-methylsulfonylbenzimidazo[1,2-*c*][1,2,3]thiadiazole **13**.

10. 3-methylsulfonylbenzimidazo[1,2-*c*][1,2,3]thiadiazol-7-sulfonyl chloride **14**.

11. (1-amino-5-bromo-1*H*-benzimidazol-2-yl)methanol 17 and (1-amino-6-bromo-1*H-*benzimidazol-2-yl)methanol **18**.

12. 3-chloro-6-bromobenzimidazo[1,2-*c*][1,2,3]thiadiazole **19**.

13. 3-chloro-7-bromobenzimidazo[1,2-*c*][1,2,3]thiadiazole **20**.

14. Composition for use in control of conditions where inhibition of carbonic anhydrase is necessary, **characterized in that** it contains effective amount of sulfonamide according to any one of claims 1 to 3.

## Patentansprüche

1. Benzimidazo[1,2-*c*][1,2,3]thiadiazol Sulfonamide der allgemeinen Formel (I) 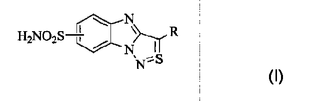 wo R für H, Cl, SCH₃, SO₂CH₃, Morpholin, Tiophenyl, N(CH₃)₂, Piperidin, N-methylpiperazin steht, und wo die Sulfonamidgruppe H₂NO₂S- in der Position 7 oder 5, 6, 8 zu finden ist, sowie auch deren pharmazeutisch zulässige Salze.

2. Benzimidazo[1,2-*c*][1,2,3]thiadiazol Sulfonamide der allgemeinen Formel (I) wie in der Anspruch 1, wo R für H, Cl, SCH₃, SO₂CH₃, Morpholin oder Tiophenyl steht und wo Sulfonamidgruppe H₂NO₂S- in der Position 7 zu finden ist, sowie auch deren pharmazeutisch zulässige Salze.

3. Verbindung nach Anspruch 2 aus foldenden Gruppen:
3-chlorobenzimidazo[1,2-*c*][1,2,3]thiadiazol-7-sulfonamid;
3-morpholinbenzimidazo[1,2-*c*][1,2,3]thiadiazol-7-sulfonamid;
3-phenylthiobenzimidazo[1,2-*c*][1,2,3]thiadiazol-7-sulfonamid;
3-methylthiobenzimidazo[1,2-*c*][1,2,3]thiadiazol-7-sulfonamid;
benzimidazo[1,2-*c*][1,2,3]thiadiazol-7-sulfonamid;
3-methylsulfonylbenzimidazo[1,2-*c*][1,2,3]thiadiazol-7-sulfonamid,
die Carboanhydrase hemmende Eigenschaften besitzen.

4. 3-chlorobenzimidazo[1,2-*c*][1,2,3]thiadiazol-7-sulfonyl chlorid **2**.

5. 3-methylthiobenzimidazo[1,2-*c*][1,2,3]thiadiazol **7**.

6. 3-methylthiobenzimidazo[1,2-*c*][1,2,3]thiadiazol-7-sulfonyl chlorid **8**.

7. Benzimidazo[1,2-*c*][1,2,3]thiadiazol **10**.

8. Benzimidazo[1,2-*c*][1,2,3]thiadiazol-7-sulfonyl chlorid **11**.

9. 3-methylsulfonylbenzimidazo[1,2-*c*][1,2,3]thiadiazol **13**.

10. 3-methylsulfonylbenzimidazo[1,2-*c*][1,2,3]thiadiazol-7-sulfonyl chlorid **14**.

11. (1-amino-5-bromo-1*H*-benzimidazol-2-yl)methanol **17** und (1-amino-6-bromo-1H-benzimidazol-2-yl)methanol **18**.

12. 3-chloro-6-bromobenzimidazo[1,2-*c*][1,2, 3]thiadiazol **19**.

13. 3-chloro-7-bromobenzimidazo[1,2-*c*][1,2,3]thiadiazol **20**.

14. Eine Rezeptur zur Steuerung von Zuständen, in denen die Inhibition der Carboanhydrase notwendig ist, die **dadurch** charakterisiert wird, daß es wirksame Mengen Sulfonamid nach einem der Ansprüche 1 bis 3 enthält.

## Revendications

1. Sulfonamides de benzimidazo[1,2-*c*][1,2,3]thiadiazole de formule générale (I) 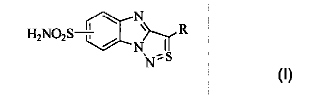 dans laquelle le radical R peut être H, Cl, SCH₃, SO₂CH₃, la morpholine, le thiophényl, N(CH₃)₂, la pipéridine, la N-méthylpiperazine, et où le groupe sulfonamide H₂NO₂S- est en position 7 ou alors dans l'une des positions 5, 6 ou 8. et se présentant en sels de qualité pharmaceutique.

2. Sulfonamides de benzimidazo[1,2-*c*][1,2,3]thiadiazole de formule générale (I) selon la revendication 1, dans laquelle le radical R peut être H, CI, SCH₃, SO₂CH₃, la morpholine ou le thiophényl et où est le groupe sulfonamide H₂NO₂S-est en position 7 et se présentant en sels de qualité pharmaceutique.

3. Composé selon la revendication 2, sélectionné dans un groupe comprenant :
3-chlorobenzimidazo[1,2-*c*][1,2,3]thiadiazol-7-sulfonamide;
3-morpholinbenzimidazo[1,2-*c*][1,2,3]thiadiazol-7-sulfonamide;
3-phenylthiobenzimidazo[1,2-*c*][1,2,3]thiadiazol-7-sulfonamide;
3-méthylthiobenzimidazo[1,2-*c*][1,2,3]thiadiazol-7-sulfonamide;
benzimidazo[1,2-*c*][1,2,3]thiadiazol-7-sulfonamide;
3-méthylsulfonylbenzimidazo[1,2-*c*][1,2,3]thiadiazol-7-sulfonamide,
qui possède des propriétés d'inhibiteur de l'anhydrase carbonique.

4. Chlorure de 3-chlorobenzimidazo[1,2-*c*][1,2,3]thiadiazol-7-sulfonyl **2**.

5. 3-méthylthiobenzimidazo[1,2-*c*][1,2,3]thiadiazole **7**.

6. Chlorure de 3-méthylthiobenzimidazo[1,2-*c*][1,2,3]thiadiazol-7-sulfonyl **8**.

7. Benzimidazo[1,2-*c*][1,2,3]thiadiazole **10**.

8. Chlorure de benzimidazo[1,2-*c*][1,2,3]thiadiazol-7-sulfonyl **11**.

9. 3-méthylsulfonylbenzimidazo[1,2-*c*][1,2,3]thiadiazole **13**.

10. Chlorure de 3-méthylsulfonylberizimidazo[1,2-*c*][1,2,3]hiadiazol-7-sulfonyl **14**.

11. (1-amino-5-bromo-1*H*-benzimidazol-2-yl)méthanol **17** et (1-amino-6-bromo-1*H*-benzimidazol-2-yl)méthanol **18**.

12. 3-chloro-6-bromobenzimidazo[1,2-*c*][1,2,3]thiadiazole **19**.

13. 3-chloro-7-bromobenzimidazo[1,2-*c*][1,2,3]thiadiazole **20**.

14. Composition destinée à être utilisée dans le contrôle des conditions où l'inhibition de l'anhydrase carboniques est nécessaire, **caractérisée en ce qu'**elle contient une quantité efficace de sulfonamide, selon l'une des revendications 1 à 3.
